(19) Europäisches Patentamt
European Patent Office
Office européen des brevets

(11) Veröffentlichungsnummer: **0 047 916 B2**

(12) # NEUE EUROPÄISCHE PATENTSCHRIFT

(45) Veröffentlichungstag der neue Patentschrift: **27.09.95**

(51) Int. Cl.⁶: **A61K 7/50**, A61K 7/08

(21) Anmeldenummer: **81106815.4**

(22) Anmeldetag: **01.09.81**

Die Akte enthält technische Angaben, die nach dem Eingang der Anmeldung eingereicht wurden und die nicht in dieser Patentschrift enthalten sind.

(54) **Körperreinigungsmittel.**

(30) Priorität: **10.09.80 DE 3033929**

(43) Veröffentlichungstag der Anmeldung: **24.03.82 Patentblatt 82/12**

(45) Bekanntmachung des Hinweises auf die Patenterteilung: **12.08.87 Patentblatt 87/33**

(45) Bekanntmachung des Hinweises auf die Entsheidung über den Einspruch: **27.09.95 Patentblatt 95/39**

(84) Benannte Vertragsstaaten: **AT BE CH DE FR GB IT LI LU NL SE**

(56) Entgegenhaltungen:
**BE-A- 840 667**
**FR-A- 2 345 142**

**SEIFEN, ÖLE, FETTE, WACHSE, Band 103, Nr. 2, 1977, Augsburg, DE. Für Tagat O bzw. Tego-Betain L7 siehe H. Janistyn: "Handbuch der Kosmetika und Riechstoffe", 3. Auflage, Band 1, "Die kosmetischen Grundstoffe", 1978, Dr. Alfred Hüthig Verlag, Heidelberg, DE., Seiten 902-911**

(73) Patentinhaber: **Th. Goldschmidt AG**
**Goldschmidtstrasse 100**
**D-45127 Essen (DE)**

(72) Erfinder: **Hüttinger, Rudolf, Dr.**
**Küppersheide 8**
**D-4300 Essen 1 (DE)**



Rank Xerox (UK) Business Services
(3.10/3.09/3.3.3)

## Beschreibung

Die Erfindung betrifft ein Körperreinigungsmittel auf der Basis einer wäßrigen Lösung eines Gemisches von

a) Betonen der allgemeinen Formel

$R^1N^{\oplus}R^2R^3(CH_2)_yCOO^{\ominus}$, I

wobei $R^1$ ein Alkylrest einer Fettsäure mit 6 bis 18 Kohlenstoffatomen oder der Rest $R^4CONH(CH_2)_x$ -, in der $R^4$ ein Alkylrest einer Fettsäure mit 6 bis 18 Kohlenstoffatomen und x = 2 oder 3 ist,

$R^2$ und $R^3$ gleich oder verschieden sind und Alkylreste mit 1 bis 4 Kohlenstoffatomen bedeuten und

y = 1, 2 oder 3 ist, wobei die Betaine im wesentlichen frei von nicht umgesetzten Fettsäureamiddialkylaminen und organisch gebundem Chlor sind,

und

b) einer oder mehreren anionischen Verbindungen aus der Gruppe Natrium- oder Ammoniumalkylethersulfat, Alkanolaminalkylethersulfat, Alkanolaminalkylsulfat, wobei die Alkylgruppe 8 bis 14 Kohlenstoffatome aufweist,

im Gewichtsverhältnis a: b von 3: 7 bis 7: 3.

Betaine der Formel I werden seit einiger Zeit und in zunehmendem Maße zur Herstellung von Duschgelen, Haarschampons, Badezusätzen und ähnlichen kosmetischen Zubereitungen verwendet.

Besonders haben sich dabei Betaine der allgemeinen Formel

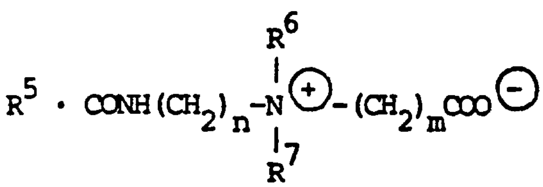

II

bewährt, wobei $R^5$ der Alkylrest einer Fettsäure mit 10 bis 18 Kohlenstoffatomen ist, $R^6$ und $R^7$ gleich oder verschieden sind und einen Alkyl- oder Hydroxyalkylrest mit 1 bis 4 Kohlenstoffatomen bedeuten, n = 2 oder 3 und m = 1, 2, 3 oder 4 ist. Die Verwendung derartiger Betaine als Badezusatzmittel ist in der DE-A-1 172 802 beschrieben.

In der Praxis hat sich nun gezeigt, daß Betaine der Formel II gelegentlich bei Personen mit sehr empfindlicher Haut Hautreizungen, insbesondere Schleimhautreizungen, verursachen können.

Es wurde deshalb ein Verfahren entwickelt DE-A- 2 926 479 nach dem die Betaine der Formel II in der Weise hergestellt werden, daß man die Quaternierungsreaktion während des gesamten Reaktionsablaufs in alkalischer Lösung, die bei 98 °C gemessen, einen pH-Wert von 7,5 bis 10 aufweist, durchführt. Die so hergestellten Betaine sind im wesentlichen frei von nicht umgesetzten Fettsäureamiddialkylaminen und organisch gebundenem Chlor. Die Produkte weisen eine verbesserte Schleimhautverträglichkeit auf.

Überraschenderweise hat sich nun gezeigt, daß sich diese gegenüber den Stand der Technik in reinerer Form erhaltenen Betaine nicht in der aus dem Stand der Technik bekannten Form durch Zusatz von anionischen Verbindungen, wie Natrium- oder Ammoniumalkylethersulfat, Alkanolaminalkylethersulfat. Alkanolaminalkylsulfat, wobei die Alkylgruppe 8 bis 14 Kohlenstoffatome aufweist, verdicken lassen.

Es ist jedoch für viele Anwendungszwecke wesentlich, daß die wäßrigen Lösungen der Betaine erhöhte Viskosität aufweisen, insbesondere z.B. für die Herstellung von Duschgelen oder Haarschampons. Dabei bereitet auch die Verdickung von Betainen der allgemeinen Formel I, bei der $R^1$ der Alkylrest einer Fettsäure mit 6 bis 18 Kohlenstoffatomen ist, die gleichen Schwierigkeiten.

Der Erfindung liegt deshalb die Aufgabe zugrunde, wäßrige Lösungen von Betainen der Formel I zu verdicken, auch wenn die Betaine dabei im wesentlichen in reiner Form vorliegen und insbesondere frei von hautreizenden Verunreinigungen sind.

Die Lösung dieser Aufgabe gelingt durch die Zusätze spezieller Glycerinmonofettsäureester. Das erfindungsgemäße Körperreinigungsmittel ist deshalb dadurch gekennzeichnet, daß es Glycerinmonofettsäureester, wobei die Fettsäurekomponente 8 bis 18 Kohlenstoffatome aufweist, mit einem Mindestanteil von 70 Gew.-% Monoester in einer Menge von 2 bis 35 Gew.-%, bezogen auf Betain, enthält.

Vorzugsweise ist das erfindungsgemäße Körperreinigungsmittel dadurch gekennzeichnet, daß es Glycerinmonolaurat mit einem Mindestanteil von 90 Gew.-% Monoester in einer Menge von 2 bis 24 Gew.-%, bezogen auf Betain, enthält.

Erfindungsgemäß ist unter dem Begriff Glycerinmonofettsäureester, deren Fettsäurekomponente 8 bis 18 Kohlenstoffatome aufweist, ein partieller Glycerinfettsäureester zu verstehen, der Mono-, Di- und Trifettsäureester enthält, wobei jedoch der Anteil an Glycerinmonofettsäureester mindestens 70 Gew.-% beträgt. Bevorzugt sind Glycerinmonofettsäureester, deren Fettsäurekomponente 8 bis 12 Kohlenstoffatome aufweist, jedoch sind auch die Fettsäureester längerkettiger Fettsäuren mit bis zu 18 Kohlenstoffatomen geeignet. In diesem Fall werden jedoch die ungesättigten Fettsäuren, insbesondere die Ölsäure, als Veresterungskomponente bevorzugt.

Unter dem Begriff Glycerinmonolaurat wird im erfindungsgemäßen Sinne nicht nur der Glyceinmonoester der Laurinsäure verstanden, sondern auch der Glycerinmonoester eines Fettsäuregemisches, welches im Mittel 12 C Atome aufweist. Insbesondere eignen sich die im der Natur vorkommenden, durch Spaltung von Fett gewonnenen Fettsäuregemische.

Der Fachmann hat es entsprechend der Lehre der Erfindung somit in der Hand, sowohl wäßrige Lösungen mäßig erhöhter Viskosität als auch hochkonsistente Gele herzustellen und somit die Zubereitung dem Verwendungszweck anzupassen. Dabei war es überraschend, daß Glycerinmonofettsäureester den wäßrigen Tensidlösungen in der erforderlichen Menge zugemischt werden konnte, da es mit Lösungen von anderen Amphotensiden häufig zu Entmischungen kommt.

Mäßig verdickte Lösungen lassen sich z.B. mit besonderem Vorteil als Badezusatzmittel oder Schampos verwenden. Gele sind wiederum als Körperreinigungsmittel für die Dusche besser geeignet. Die Zubereitungen lassen sich auch als Flüssigseifen verwenden, wobei die Viskosität der Seifenlösung der dosierenden Abgabevorrichtung angepaßt werden kann.

Es hat sich überraschenderweise zusätzlich gezeigt, daß durch den Zusatz von Glycerinmonofettsäureestern die Schleimhautverträglichkeit wäßriger Betainlösungen noch weiter verbessert wird, so daß Glycerinmonofettsäureester nicht nur verdickend, sondern auch verträglichkeitsverbessernd wirken. Außerdem zeigt sich ein von der Konzentration der zugesetzten Glycerinmonofettsäureester abhängiger deutlicher Rückfettungseffekt.

Die erfindungsgemäßen Zubereitungen lassen sich mit den üblichen Konfektionierungsmitteln, wie z.B. Farbstoffen, Parfümierungsölen, Konservierungsmitteln und zusätzlichen anderen hautpflegenden Substanzen, versetzen und in die von dem Kunden gewünschte verkaufsgerechte Form bringen.

In der folgenden Tabelle sind verschiedene Rezepturen und deren Viskositäten in mPas angegeben.

Tabelle I

| Betain | anionische Komponente | | | Wasser | Monoglycerid | | | Viskosität der Zubereitung mPas, 20°C |
|---|---|---|---|---|---|---|---|---|
| Gew.-% und Typ | Typ | Kohlenstoffanzahl des Alkylrestes | Gew.-% | Gew.-% | Kohlenstoffanzahl des Fettsäurerestes | Gehalt an Monoglycerid | Gew.-% | |
| 7,1 I | A | 12 | 7,0 | 84,5 | 12 | 90 % | 1,4 | 50.000 |
| 8,5 I | A | Gemisch aus 12 bis 14 | 5,0 | 84,9 | 12 | 90 % | 1,6 | 21.000 |
| 7,1 I | A | Gemisch aus 12 bis 16 | 5,8 | 85,7 | 12 | 90 % | 1,4 | 24.000 |
| 7,1 I | A | 12 | 7,0 | 84,5 | Gemisch aus 8 bis 18* | 80 % | 1,4 | 47.000 |
| 7,1 I | A | 12 | 7,0 | 84,5 | Gemisch aus 8 bis 18** | 90 % | 1,4 | 30.000 |
| 7,1 I | A | 12 | 7,0 | 84,5 | 18*** | 90 % | 1,4 | 46.000 |
| 7,1 I | A | 12 | 7,0 | 84,5 | 12 | 70 % | 1,4 | 20.000 |
| 7,1 I | B | 12 | 12,5 | 79,0 | 12 | 90 % | 1,4 | 45.000 |
| 4,5 II | A | 12 | 9,8 | 84,9 | 12 | 90 % | 0,8 | 90.000 |

In der Tabelle I bedeuten:

Betain Typ I

entspricht der Formel I, wobei die Indizes folgende Bedeutung haben:
$R^4$ = Alkylrest mit 6 bis 18 Kohlenstoffatomen
$R^2$ und $R^3$ = Methylreste
x = 3
y = 1

Betain Typ II

entspricht der Formel I, wobei die Indizes folgende Bedeutung haben:
$R^1$ = Alkylrest mit 14 bis 16 Kohlenstoffatomen
$R^2$ und $R^3$ = Methylreste
y = 1
A = Natriumalkylethersulfat
B = Triethanolaminlaurylsulfat
* = Fettsäurekomponente aus Kokosöl
** = Fettsäurekomponente aus gehärtetem Kokosöl
*** = Fettsäurekomponente Ölsäure

## Patentansprüche

**1.** Körperreinigungsmittel auf der Basis einer wäßrigen Lösung eines Gemisches von
a) Betainen der allgemeinen Formel

$$R^1N^+R^2R^3(CH_2)_yCOO^-$$

wobei $R^1$ ein Alkylrest einer Fettsäure mit 6 bis 18 Kohlenstoffatomen oder der Rest $R^4CONH(CH_2)_x$ -, in der $R^4$ ein Alkylrest einer Fettsäure mit 6 bis 18 Kohlenstoffatomen und x=2 oder 3 ist, $R^2$ und $R^3$ gleich oder verschieden sind und Alkylreste mit 1 bis 4 Kohlenstoffatomen bedeuten und y = 1, 2 oder 3 ist, wobei die Betaine im wesentlichen frei von nicht umgesetzten Fettsäureamiddialkylaminen und organisch gebundem Chlor sind, und
b) einer oder mehreren anionischen Verbindungen aus der Gruppe Natrium oder Ammoniumalkylethersulfat, Alkanolaminalkylethersulfat, Alkanolaminalkylsulfat, wobei die Alkylgruppe 8 bis 14 Kohlenstoffatome aufweist,
im Gewichtsverhältnis a: b von 3: 7 bis 7: 3, dadurch gekennzeichnet, daß es Glycerinmonofettsäureester, wobei die Fettsäurekomponente 8 bis 18 Kohlenstoffatome aufweist, mit einem Mindestanteil von 70 Gew.-% Monoester in einer Menge von 2 bis 35 Gew.-%, bezogen auf Betain, enthält.

**2.** Körperreinigungsmittel nach Anspruch 1, dadurch gekennzeichnet, daß es Glycerinmonolaurat mit einem Mindestanteil von 90 Gew.-% Monoester in einer Menge von 2 bis 24 Gew.-%, bezogen auf Betain, enthält.

## Claims

**1.** Body cleanser based on an aqueous solution of a mixture of
a) betaines of the general formula

$$R^1N^{\oplus}R^2R^3(CH_2)_yCOO^{\ominus}$$

wherein $R^1$ is an alkyl radical of a fatty acid with 6 to 18 carbon atoms or the radical $R^4CONH(CH_2)_x$-, in which $R^4$ is an alkyl radical of a fatty acid with 6 to 18 carbon atoms and x = 2 or 3,
$R^2$ and $R^3$ are identical or different and denote alkyl radicals with 1 to 4 carbon atoms and y = 1, 2 or 3, the betaines being essentially free from unreacted fatty acid amide dialkylamines and organically bound chlorine, and
b) one or more anionic compounds from the group consisting of sodium or ammonium alkyl ether sulphate, alkanolamine alkyl ether sulphate and alkanolamine alkylsulphate, the alkyl group having 8 to 14 carbon atoms,

in a weight ratio a : b of 3 : 7 to 7 : 3, characterized in that it contains glycerol fatty acid monoesters, the fatty acid component having 8 to 18 carbon atoms, with a minimum proportion of 70% by weight of monoester, in an amount of 2 to 35% by weight, based on betaine.

**2.** Body cleanser according to claim 1, characterized in that it contains glycerol monolaurate, with a minimum proportion of 90% by weight of monoester, in an amount of 2 to 24% by weight, based on betaine.

**Revendications**

**1.** Agent de lavage corporel à base d'une solution aqueuse d'un mélange

a) de bétaïnes de formule générale

$R^1N^{\oplus}R^2R^3(CH_2)_YCOO^{\ominus}$

dans laquelle $R^1$ est un résidu alkyle d'un acide gras ayant de 6 à 18 atomes de carbone, ou le radical $R^4CONH(CH_2)_x$-, dans lequel $R^4$ est un résidu alkyle d'un acide gras ayant de 6 à 18 atomes de carbone, et x = 2 ou 3,

$R^2$ et $R^3$ sont identiques ou différents et représentent chacun des radicaux alkyle ayant de 1 à 4 atomes de carbone, et

y = 1, 2 ou 3, où les bétaines sont pour l'essentiel exemptes d'(amide d'acide gras)-dialkylamines n'ayant pas réagi et de chlore organiquement lié,

et

b) d'un ou plusieurs composés anioniques du groupe comprenant les alkyléthersulfates de sodium ou d'ammonium, les alkyléthersulfates d'alcanolamine, les alkylsulfates d'alcanolamine, le groupe alkyle ayant de 8 à 14 atomes de carbone,

selon un rapport pondéral a : b de 3 : 7 à 7 : 3, caractérisé en ce qu'il contient, en une quantité de 2 à 35 % en poids par rapport à la bétaïne, un monoester d'acide gras du glycérol contenant au moins 70 % en poids de monoester, le composant acide gras ayant de 8 à 18 atomes de carbone.

**2.** Agent de lavage corporel selon la revendication 1, caractérisé en ce qu'il contient en une quantité de 2 à 24 % en poids par rapport à la bétaïne du monolaurate de glycérol contenant au moins 90 % en poids du monoester.